# EUROPEAN PATENT APPLICATION

(11) **EP 4 056 674 A1**
(43) Date of publication of application: **14.09.2022**
(21) Application number: 21162427.5
(22) Date of filing: 12.03.2021
(51) Int. Cl.: C12N 1/14, E04B 1/14, E04C 2/16, E04C 2/38, E04B 2/00, E04B 5/02, A01G 18/00, E04B 1/80, E04B 1/88

(54) **METHOD OF MANUFACTURING A CONSTRUCTION AND/OR INSULATION MATERIAL**

(71) Applicant: FS-Insulation B.V., 3011 TA Rotterdam (NL)
(72) Inventor: van Driel, Roland, 7827 TB Emmen (NL); Borra, Hans Antonius, 3707 TB Zeist (NL)
(74) Representative: De Vries & Metman

(57) **Abstract**

The invention relates to a method of manufacturing a construction and/or insulation material comprising the steps of providing a fungus and a substrate, introducing or preparing a mixture of the fungus and the substrate in a mold, allowing the fungus to grow to form a network of hyphae through the mixture to form a mycelium composite, taking the composite from the mold, and shredding the composite to chunks.

## Description

The invention relates to a method of manufacturing a construction and/or insulation material comprising the steps of providing a fungus and a substrate, introducing or preparing a mixture of the fungus and the substrate in a mold, allowing the fungus to grow to form a network of hyphae through the mixture to form a mycelium composite, and taking the composite from the mold. The invention also relates to a construction and/or insulation material, such as a plate, a panel, a load-bearing element for frame construction, or a insulation layer or wall.

WO 2008/073489 relates to a composite material that is comprised of a substrate of discrete particles and a network of interconnected mycelia cells bonding the discrete particles together. The composite material is made by inoculating a substrate of discrete particles and a nutrient material with a preselected fungus. The fungus digests the nutrient material over a period of time sufficient to grow hyphae and to allow the hyphae to form a network of interconnected mycelia cells through and around the discrete particles thereby bonding the discrete particles together to form a self-supporting composite material.

WO 2018/014004 relates to forming fungal materials and fungal objects from those fungal materials, the method comprising the steps of growing a first fungal tissue in contact with a nutritive vehicle; supplying a porous material in contact with said first fungal tissue; directing growth of said fungal tissue through said porous material such that a portion of said fungal tissue comprises a first fungal material having first fungal hyphae; optionally incorporating composite material; directing a change in the composition or growth pattern of at least some of said first fungal hyphae; separating at least a portion of the first fungal material from said nutritive vehicle; obtaining a second fungal material having second fungal hyphae; and forming a fungal object by encouraging fused growth between said first fungal material and said second fungal material and optionally incorporating composite material.

It is an object of the present invention to provide an improved method of manufacturing a construction and/or insulation material.

To this end, the method comprises the step of shredding the mycelium composite to chunks. In an embodiment, the method comprises the step of drying the chunks, e.g. by means of heat and/or vacuum e.g. in a drying chamber. An embodiment comprises the steps of introducing the (dried) chunks into an enclosure, such as a mold, and allowing the fungus to grow to form a network of hyphae around and/or through the chunks to form a further composite.

The chunks according to the present invention are suitable as or for use in a construction and/or insulation material that, compared to the initial composite, comprises (interstitial) cavities and that has significantly improved insulation properties. Further, the chunks facilitate accelerated manufacture of further composites, in that the chunks can be dried at a first location and/or a first point in time and used as a raw material for a further mycelium composite at another location and/or a later point in time. Thus, the further mycelium composite already comprises for at least a substantial part a dried mycelium composite and thus requires less or no drying.

In an embodiment, the method comprises the steps of introducing or preparing a mixture of the chunks with (additional) fungus and/or (additional) substrate in an enclosure, and allowing the fungus to grow to form a network of hyphae around and/or through the chunks to form a (further) mycelium composite.

The fresh mixture further reinforces the further composite and/or facilitates forming one or more flat of substantially flat surface on the further composite. In an embodiment, the additional fungus and substrate are added in an amount of 10 wt% to 40 wt% of the total of chunks and added fungus and substrate.

In an embodiment the enclosure is part of or defined by a roofing, flooring or wall panel, the fungus forms a network of hyphae into the walls of the enclosure, and the further composite is dried while it remains in the enclosure of the panel.

Thus, the roofing, flooring, or wall panel serves as a formwork or mold for the mycelium composite and remains (a major) part of the actual prefab panel. By manufacturing a prefab construction element in this way, the further composite adapts in shape and bonds to the panel and contributes to the strength, the stiffness and to the insulating properties of the prefab construction element.

In an embodiment, the chunks have an average diameter in a range from 0,5 to 10 centimeters, preferably 1 to 8 centimeters, preferably 2 to 7 centimeters. In a refinement, at least 80 wt%, preferably at least 90 wt% of the chunks have a diameter in that range.

Size and size distribution of the chunks can be better controlled if the composite is shredded after colonization is completed, i.e. after the hyphae have spread and have provided coherence throughout the composite, including its center.

Establishing that colonization is completed can be done e.g. by taking samples, my measuring electrical conductivity, and/or empirically, e.g. if the same mold and mixture are used, progress and completion can be ascertained by (invasively) checking the degree of colonization after increasing time intervals.

In an embodiment, shredding involves breaking and/or tearing, which mechanisms introduce little or no compression of the composite.

In an embodiment, the fungus in the chunks is stopped, in particular inactivated or killed, typically during or after drying of the chunks or the further composite, by means of heating, reduced pressure, freezing, radiation and/or drying.

Suitable substrates for creating mycelium composite include wood materials, e.g. particles, such as saw dust and wood shavings, and materials from grain, maize, rice, or hemp.

Materials that can be added, e.g. up to a total amount of 40 wt% of the substrate in total, to the substrate include vegetable materials such as cucumber, peppers, grass, reed, beer broth, potato steam peel, root pulp and used growing substrates from greenhouses. Other examples are polystyrene, plastics, and cardboard materials, as well as inorganic materials such as perlite and vermiculite.

The fungus digests the nutrient components in the substrate over a period of time sufficient to grow hyphae and to allow the hyphae to form a network of interconnected mycelia cells through and around the discrete particles in the substrate thereby bonding the discrete particles together to form a mycelium composite and, where applicable, bonding the composite to the walls of the enclosure.

The at least one fungus is preferably a white rot fungus and preferably one that grows relatively quickly and/or is able to accept materials that are strange to its habitat.

In an embodiment, the fungus or at least one of the fungi is selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

Suitable nutrients include sugar, oatflakes, flour, rejected food, and human and animal hair.

In an embodiment, during at least part of the growing of the hyphae, the enclosure is covered, in particular to prevent moisture from escaping, and/or the temperature of the mixture is maintained in a range from 15 to 24 degrees Celsius.

The invention further relates to a construction and/or insulation element, such as a plate, a panel, a load-bearing element for frame construction, or a insulation layer or wall, comprising chunks of mycelium composite, preferably chunks obtained with the method according to any one of the preceding claims.

In an embodiment, the construction and/or insulation material, has a thermal conductivity, lambda (λ), of 0,037 W/mK or less, preferably 0,032 W/mK or less.

Many roofing, flooring, or wall panels have a thickness of at least 20 cm, often 25 centimeters or more, and are provided with rock wool or glass wool to provide insulation. By using a relatively thick layer of the chunks of mycelium composite, preferably at least 15 centimeters, preferably at least 20 centimeters, the panel exhibits sufficient heat and noise insulation, without requiring a further material, such as rock wool or glass wool.

In an embodiment, the panel is made of compressed mycelium board, wood, fiberboard, plywood, or other cellulose based material.

The invention also relates to a prefab construction element, such as a load-bearing element, for frame construction, such as wood frame construction, comprising a roofing, flooring or wall panel, which panel comprises an enclosure, which enclosure contains the mycelium composite described above, wherein a network of hyphae has formed through the material and into the walls of the enclosure. In an embodiment, the mycelium permanently binds the mycelium composite to the panels.

In an embodiment, the construction element is a panel that comprises rafters dividing the enclosure in sections, and roof slabs and/or vertical battens fixed, e.g. nailed, to the panel and optionally horizontal battens fixed to the vertical battens.

In another embodiment, the ratio of the weight of the panel and the weight of the mixture, i.e. the weight of the panel divided by the weight of the mixture is smaller than 0,6, preferably smaller than 0,5, preferably smaller than 0,45 and/or the rafters have a width less than 25 mm, preferably less than 20 mm.

Within the framework of the present invention, the verb "to shred", includes tearing, breaking, crumbling, grinding, cutting and combinations thereof. It is preferred that, during shredding, compression of the composite is avoided or at least limited.

The invention will now be explained in more detail with reference to the figures, which schematically show an embodiment according to the present invention.
Figure 1 is a photo of chunks of mycelium composite according to the present.
Figure 2 show panels wherein a mixture of the chunks, shown in Figure 1, substrate and fungus has been introduced in the enclosure.
Figure 3 is an isometric view of a prefab construction element according to the present invention.

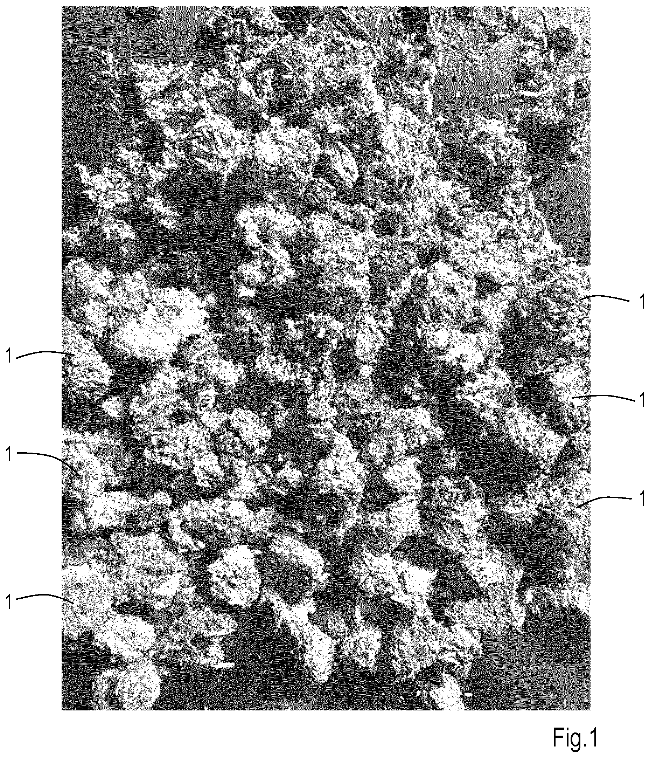

Figure 1 shows chunks of a mycelium composite in accordance with the present invention. The chunks were prepared by homogenously mixing substrate, e.g. wood shavings, straw, and grass, and a 1% solution of hydrogen peroxide. After 30 to 60 minutes rest, the substrate was mixed again, primarily to allow any remaining peroxide to escape. Next, the substrate was mixed homogenously with a fungus (inoculum), for instance Pleurotus ostreatus, optionally at least one nutrient, such as oatflakes, and water, which mixture was introduced into a mold, and covered, e.g. with an impermeable foil or tarpaulin. The temperature of the mixture was maintained in a range from 15 to 24°C, for example at 20°C. The fungus was allowed to grow for a period in a range from 50 to 120 hours, preferably in a range from 70 to 110 hours, for example 100 hours, to form a network of hyphae through the mixture.

When the mycelium composite was considered at or near optimum, in terms of coherence, the mycelium composite was removed from the mold and shredded to chunks having an average diameter of about 5 centimeters, with 80 wt% of the particles having a diameter in a range from 3 to 8 centimeters.

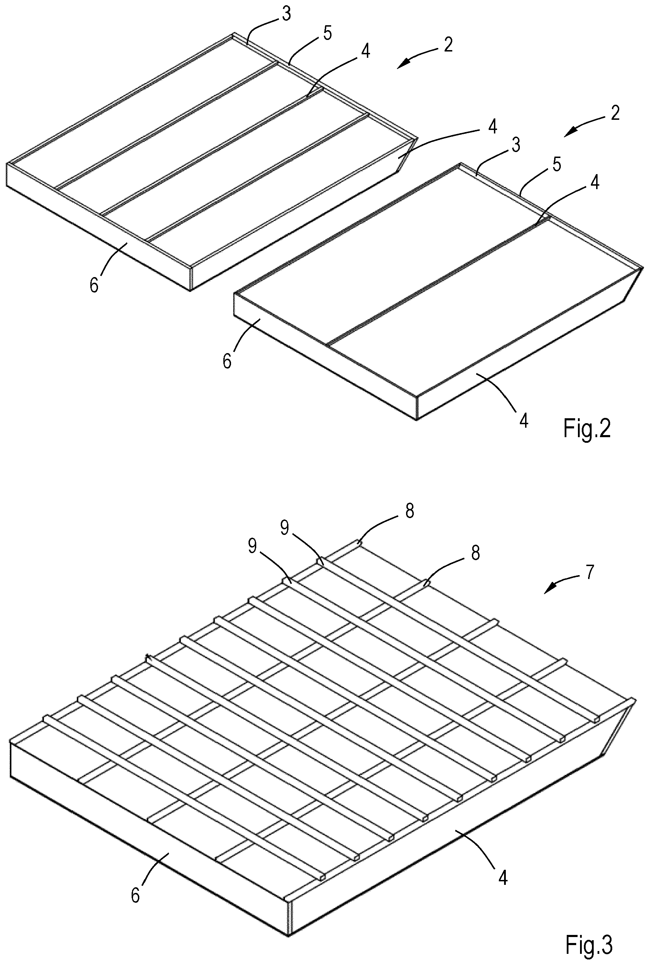

The chunks were dried through forced convection of air at ambient temperature. The dried chunks were mixed with 20 wt% of a fresh mixture of substrate and fungus, identical to the mixture from which chunks were formed. This mixture of chunks, substrate, and fungus was introduced as bulk into the enclosure(s) of a roofing panel as shown in Figure 2.

Figure 2 shows two examples of a roofing panel 2, each of which has an enclosure 3 defined by walls made e.g. of wood or fiberboard and, in this example, comprising side walls 4, upper and lower end walls 5, 6, and a bottom wall (hidden from view by the mixture). The upper end wall 5 forms a headboard that is at an inclination and that, once the panel is installed on a roof, forms the apex of the roof (inner) construction and supports e.g. a ridge beam and/or ridge tiles. The lower end wall 6 forms a gutter board.

The side walls are in effect rafters that play a major role in providing strength and stiffness to the panel 2. One of the panels shown in Figure 2 comprises a total of five rafters 4, two on the sides (co)defining the enclosure 3 of the panel 1 and three inside the enclosure, dividing the enclosure into four sections. The other panel 2 according to the present invention comprises a total of three rafters 5, two sides (co)defining the enclosure 3 of the panel 2 and one inside the enclosure dividing the enclosure into two sections.

The fungus in the enclosure of the panel was allowed to grow, to form a network of hyphae around and/or through the chunks to form a mycelium composite and into the walls of the enclosure. When the mycelium composite was considered at or near optimum, in terms of strength, stiffness and durability in a dried state, the fungus was killed by heating and drying the prefab construction element and the mycelium composite in it.

Figure 3 shows a prefabricated construction element 7 wherein the panel 2 comprises, in addition to the rafters 4, vertical battens 8 fixed to the panel 2 and optionally horizontal battens 9 fixed to the vertical battens 8, thus ready to be installed in a wood frame building.

In comparison to a traditional panel having the same external dimensions, the prefabricated element according to this example equally fulfills official requirements (building codes) for strength, stiffness, and insulation, but require at least 45% less wood.

The invention is not restricted to the above-described embodiments, which can be varied in a number of ways within the scope of the claims, and, for instance, applies similarly advantageously in construction elements for floors and walls. Also, the dried chunks, after inactivating the fungus, can be used an insulating material, e.g. by introducing (blowing) the chunks into the cavity of a cavity wall.

## Claims

1. Method of manufacturing a construction and/or insulation material comprising the steps of
providing a fungus and a substrate,
introducing or preparing a mixture of the fungus and the substrate in a mold,
allowing the fungus to grow to form a network of hyphae through the mixture to form a mycelium composite,
taking the composite from the mold, and
shredding the composite to chunks.

2. Method according to claim 1, comprising the step of drying the chunks.

3. Method according to claim 1 or 2, comprising the steps of introducing the chunks into an enclosure and
allowing the fungus to grow to form a network of hyphae around and/or through the chunks to form a further composite.

4. Method according to any one of the preceding claims, comprising the steps of preparing or introducing a mixture of the chunks, fungus and substrate in an enclosure, and
allowing the fungus to grow to form a network of hyphae around and/or through the chunks to form a mycelium composite.

5. The method according to claim 4, wherein the enclosure is part of or defined by a roofing, flooring or wall panel,
the fungus forms a network of hyphae into the walls of the enclosure, and
the further composite is dried while it remains in the enclosure of the panel.

6. Method according to any one of the preceding claims, wherein the chunks have an average diameter in a range from 0,5 to 10 centimeters, preferably 1 to 8 centimeters, preferably 2 to 7 centimeters.

7. Method according to claim 6, wherein at least 80%, preferably at least 90% of the chunks have a diameter in that range.

8. Method according to anyone of the preceding claims, wherein the composite is shredded after colonization is completed.

9. Method according to anyone of the preceding claims, wherein shredding involves breaking and/or tearing.

10. Method according to any one of the preceding claims, wherein the growth of the fungus in the chunks is stopped by means of heating, reduced pressure, freezing, radiation and/or drying.

11. Method according to any one of the preceding claims, wherein the fungus or at least one of the fungi is selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

12. Construction and/or insulation material, such as a plate, a panel, a load-bearing element for frame construction, or a insulation layer or wall, comprising chunks of mycelium composite, preferably chunks obtained with the method according to any one of the preceding claims.

13. Construction and/or insulation material according to claim 12, having a thermal conductivity, lambda (λ), of 0,037 W/mK or less, preferably 0,032 W/mK or less and/or a specific weight in a range from 100 to 200 kg/m3, preferably in a range from 120 to 180 kg/m3, and/or a porosity in a range from 10% to 50%, preferably 12% to 40%, preferably 15% to 30%.

14. Construction and/or insulation material according to claim 12 or 13, wherein the chunks have an average diameter in a range from 0,5 to 10 centimeters, preferably 1 to 8 centimeters, preferably 2 to 7 centimeters and/or at least 80 wt%, preferably at least 90 wt% of the chunks have a diameter in that range.

15. Prefabricated construction and/or insulation element, such as a plate, a panel, a load-bearing element for frame construction, or a insulation layer or wall, comprising a material according to any one of the claim 12-14.
